Europäisches Patentamt

European Patent Office

Office européen des brevets

(19)

(11) Publication number: 0 355 325
A1

## (12) EUROPEAN PATENT APPLICATION

(21) Application number: 89111819.2

(22) Date of filing: 29.06.89

(51) Int. Cl.⁴: **F16J 15/02** , **B01D 63/02** , **B01D 65/00**

(30) Priority: 07.07.88 SE 8802542

(43) Date of publication of application:
28.02.90 Bulletin 90/09

(84) Designated Contracting States:
BE CH DE ES FR GB IT LI NL

(71) Applicant: GAMBRO DIALYSATOREN GMBH &
CO. KG
Postfach 1323
D-7450 Hechingen(DE)

(72) Inventor: **Spranger, Kurt**
**Obere Strasse 43**
**D-7403 Ammerbuch 1(DE)**
Inventor: **Antoni, Roland**
**Jahnstrasse 8**
**D-7450 Hechingen-Boll(DE)**
Inventor: **Lutterbeck, Joachim, Dr.-Ing.**
**Kornbühlstrasse 24**
**D-7450 Hechingen(DE)**
Inventor: **Ott, Gerd**
**Schweizer Gasse 18**
**D-7453 Burladingen 6(DE)**
Inventor: **Raabe, Herbert**
**Beethovenweg 4**
**D-7452 Haigerloch-Stetten(DE)**
Inventor: **Volm, Josef**
**Hauptstrasse 7**
**D-7452 Haigerloch-Owingen(DE)**

(74) Representative: **Boberg, Nils Gunnar Erik**
**Gambro AB Patent Department Box 10101**
**S-220 10 Lund(SE)**

(54) **A seal comprising a ring of a resilient material intended to be pressed between two parallel, preferably flat sealing surfaces.**

(57) A seal is described comprising a ring (17) of a resilient material intended to be pressed between two parallel, preferably flat sealing surfaces (13,16).

Said seal is preferably intended to be a part of a filtration and/or diffusion device, comprising a bundle (1) of hollow fibers arranged longitudinally within a housing (2) with the fiber ends embedded in two end walls (3) and with one sealing ring (17) being positioned between the outside (13) of one or both said end walls (3) and a lid (14) having an inlet and/or an outlet (15) for a liquid intended to flow through the hollow fibers of the bundle.

Said device is preferably intended to be used for dialysis, hemofiltration or hemodiafiltration.

## Fig. 1

# A SEAL COMPRISING A RING OF A RESILIENT MATERIAL INTENDED TO BE PRESSED BETWEEN TWO PARALLEL, PREFERABLY FLAT SEALING SURFACES

## TECHNICAL FIELD

The present invention relates to a seal comprising a ring of a resilient material intended to be pressed between two parallel, preferably flat sealing surfaces.

The seal according to the present invention is preferably intended to be a part of a filtration and/or diffusion device according to for instance any of the Swedish patents (SE appl 87.03366-8) and 454 847 (SE appl 87.03368-4), i e a device comprising a bundle of hollow fibers arranged longitudinally within a housing with the fiber ends embedded in two end walls and with the sealing ring being positioned between the outside of one or both said end walls and a lid having an inlet and/or an outlet for a liquid intended to flow through the hollow fibers of the bundle.

In connection with devices of this kind, special advantages will be achieved by using seals according to the present invention. For the man skilled in the art it will, however, be ovbious that seals of this kind also may be used advantageously for many other purposes.

## BACKGROUND ART

In the above mentioned patents, some different filtration and/or diffusion devices are disclosed, all having a sealing ring in the form of an O-ring arranged between an end wall and a cup-shaped lid. In practice it has, however, been found that it is very difficult by means of such an O-ring to provide exactly the desired tightening pressure. This is especially true when the lid is to be welded to the housing of the device. In order to get the desired tightening pressure, the lid has to be placed in a special position as regards the housing within very narrow limits.

## DISCLOSURE OF INVENTION

One object of the present invention is to solve the above problem which we believe is common for many different technical fields. The problem is solved in that the sealing ring is given a special form characterized in that the dimension of the ring perpendicularly to the two parallel surfaces is larger than the dimension parallel to said surface and that it is given such a form that it is bent in a certain direction in its sealing position.

Alternatively it may be said that the ring is given such a form that it by the compression quickly reaches a knicking or bending position from which the compression forces increases slowly compared with the initial state of the compression, eventually reaching a position from which the compression forces once again increases more rapidly.

By such a construction you may move the lid as regards the housing in a device of the above described kind within wide limits and still have small changes of the compression forces as will be described more in detail in the following in connection with the description of the drawings.

Preferably, the seal according to the invention includes, beside said two parallel sealing surfaces, also a third sealing surface, which is perpendicular to the other sealing surfaces and encloses the sealing ring. By such a construction a liquid under pressure within the ring will press the ring against said third sealing surface providing an improvement of the tightness which is further improved in that the same liquid pressure will provide an increased sealing pressure also against the two first mentioned sealing surfaces.

If the ring is given such a form that it is directing a concave surface against said third sealing surface in its sealing position, the advantage is gained that you get a parallel sealing along two parallel lines at said third sealing surface in parallel to the two opposite sealing lines against the two above first mentioned sealing surfaces.

Preferably the ring is given a form of a boomerang or a C-form before it is compressed. The concave side of the ring being directed outwardly against said third sealing surface. In this way you get a convex side of the ring directed inwardly the center of the ring. If this convex side of the ring is being exposed for an inner pressure, it will be more or less flattened which is of an advantage especially when the liquid within the ring is a perishable liquid such as blood. This tendency may be further strengthened by an indentation on the convex side of the ring, i e an indentation having such a form that said side of the ring will get an essentially flat form perpendicular to the two first mentioned sealing surfaces in the sealing position of the ring.

Special advantages are gained, if the seal according to the present invention is a part of a filtration and/or diffusion device, for instance a dialyzer, comprising a bundle of hollow fibers arranged longitudinally within a housing with the fiber ends embedded in two end walls and with the sealing ring being positioned between the outside of one or both said end walls and a lid having an inlet and/or outlet for a liquid intended to flow through the hollow fibers of the bundle. As mentioned above the seal can in such a construction be given such a form that it exercises a very gently influence on the liquid, for example blood, passing the seal. In comparison with a normal O-ring you don't get the typical wedge-formed gaps above and below the ring where the ring contacts the two first mentioned sealing surfaces.

Special advantages are also gained when the above discussed device is provided with a lid or lids which has/have a cup-form with a flange covering the outside of the ends of the housing and which may be either welded or glued to the housing or provided with a screw connection with the housing. Thanks to the invention such a lid may be manufactured with larger tolerances and this is especially true for lids intended to be welded to the housing. Normally a lid intended to be welded to the housing has to be made within very narrow tolerances due to the fact that the welding machine restricts the possibilities to place lids of different dimensions in different positions in order to provide a desired tightening pressure.

The resilient ring is preferably made of rubber or a similar elastic material. Other materials are, however, also possible, if they may be given such a form and resiliency that you get the above described functions.

BRIEF DESCRIPTION OF DRAWINGS

Fig 1 shows a longitudinal section through a filtration and/or diffusion device provided with a seal according to the present invention. The device is provided with a lid which is welded or glued to a matching housing.

Fig 2 shows a longitudinal section through a modified filtration and/or diffusion device having a lid which is attached to a matching housing with a screw connection.

Fig 3a-3c are showing a transverse section through a sealing ring according to the present invention exposed to three different pressures.

Fig 4a-4c are showing three transverse sections through a modified sealing ring exposed to the same pressures.

Fig 5, finally, is showing the deformation force exercised on a normal O-ring and a sealing ring according to the present invention, respectively, when said force is increased from zero to a maximum.

PREFERRED EMBODIMENTS OF THE INVENTION

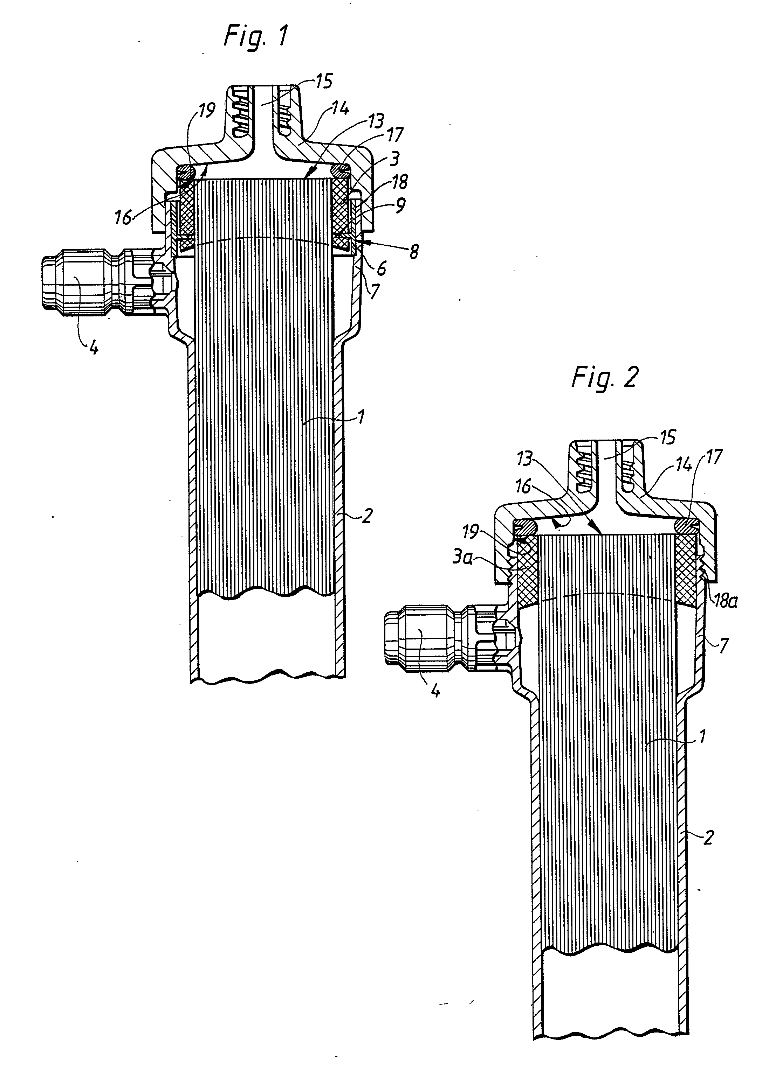

Fig 1 shows one end of a filtration and/or diffusion device of the kind disclosed and claimed in the above mentioned Swedish patent 454 847 (SE appl 87.03368-4), but provided with a sealing ring 17 according to the present invention. The device consists of a bundle of hollow fibers 1 arranged in a housing 2 with the ends embedded in a potting material providing an end wall 3. The ends of the fibers have been opened by a transverse cut providing an end surface 13 as described in for instance US patent 4 689 191. The device is provided with an inlet and/or outlet 4 and a second outlet and/or inlet 15 for first and second liquids, respectively. Normally, the other end of the device is given an identical form. However, if the device is intended to be used only for filtration you don't need a second nipple 4.

The nipple 4 serving as an inlet or an outlet is arranged on an enlarged part 7 of the housing 2. Within said enlarged part 7 there is arranged in a groove 8 a ring 6 with an inner flange 9, serving to reduce the contact between the end wall 3 and the housing 2 as described more in detail in the above mentioned Swedish patent. For that purpose the ring 6 is made of a material having a coefficient of addition as regards the material of the end wall which is less than the coefficient of addition of the housing as regards the same material of the end wall.

The inlet and/or outlet 15 is arranged in a lid 14 which is attached to the enlarged part 7 of the housing 2 by a welding 18. Alternatively, it may be attached by glue.

The two above first mentioned parallel sealing surfaces are provided by on one hand the end surface 13 of the fiber bundle 1 and on the other hand by the inside 16 of the lid 14. The third sealing surface is designated 19 and provided by the inside of the flange of the lid 14.

Fig 2 shows a more conventional filtration and/or diffusion device modified in accordance with the present invention. Many details of fig 2 are essentially identical to corresponding details of fig 1 and have therefor been given the same reference numerals. Consequently, the device according to fig 2 comprises a bundle of hollow fibers 1 within a housing 2 with an enlarged part 7 to which a lid 14 is attached. The attachment is, however, not made by glue or welding but by means of a screw connection 18a. The end surface of the fiber bundle has been designated 13 and the fiber ends have been opened as described above. The device includes in the same way as the device described above inlets and/or outlets 4 and 15 and a sealing ring 17 according to the present invention. An end wall 3a made by a potting material has contrary to the end wall 3 a strong attachment to the inner wall of the enlarged part 7 of the housing 2.

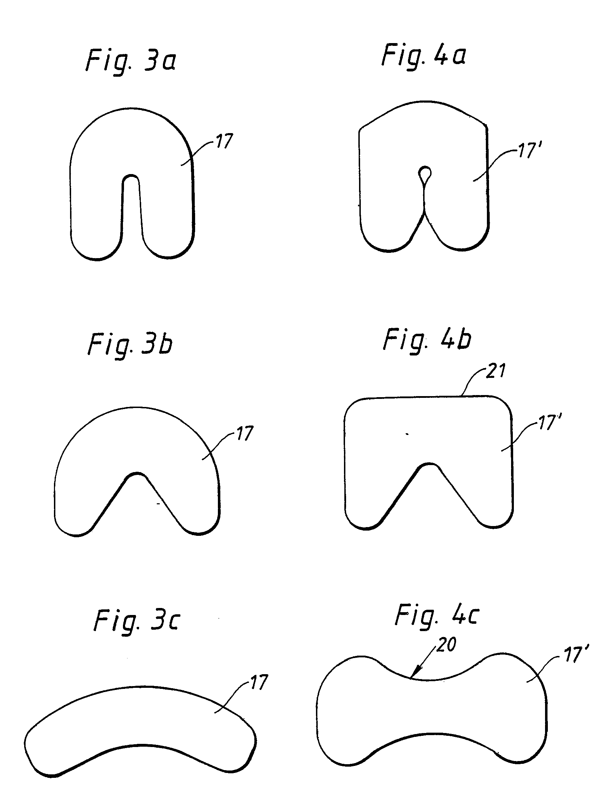

In order to explain the function of the sealing ring 17 reference is made to fig 3a-3c, 4a-4c and fig 5. In unpressed condition the ring may have the section shown in either fig 3c or fig 4c, the upper side being in both cases intended to be directed inwardly against the liquid passing trough the inlet and/our outlet 15.

Fig 3b and 4b are showing the same transverse sections through the sealing ring in an ideal sealing position. The flat surface 21 shown in fig 4b of the modified sealing ring 17' according to fig 4a-4c is provided thanks to the indentation 20 shown in fig 4c.

By a further compression you will eventually reach the positions shown in fig 3a and 4a, still providing a good seal. However, after that, a further compression will rapidly increase the deformation force in an unacceptable way.

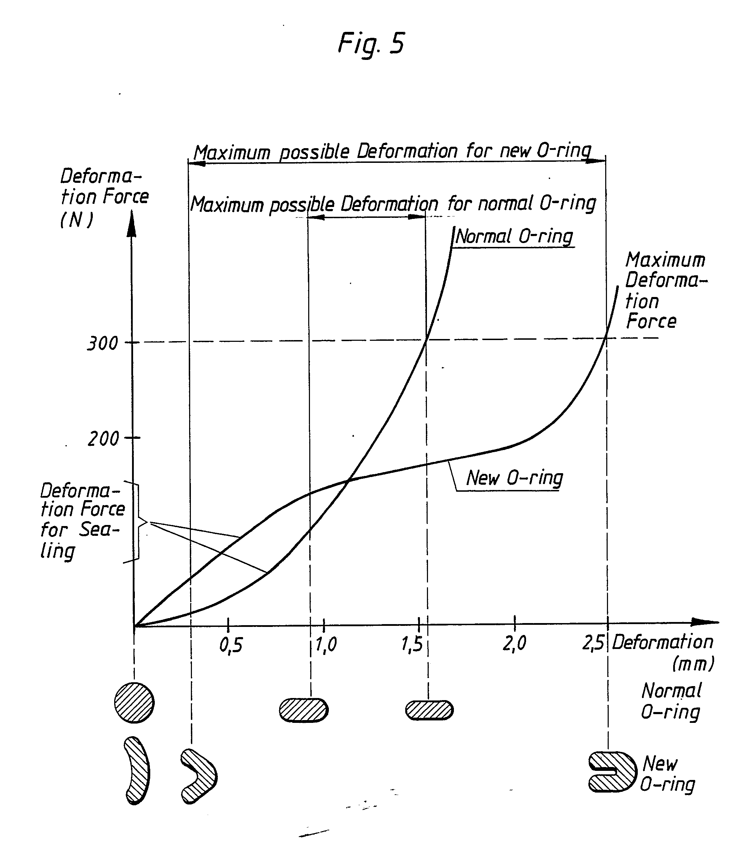

Fig 5 shows how the deformation force is changed in response to the deformation for on one hand a normal O-ring and on the other hand a sealing ring according to the present invention. The comparison is given in the form of an example. By using different materials and different dimensions and forms the figures in the fig 5 will of course vary. In the example shown, a normal O-ring may be used with a deformation of between 0,9 and 1,6 mm. A sealing ring according to the present invention reaches, however, an acceptable sealing pressure much more rapidly, i e after about 0,3 mm and the deformation force is still below the acceptable 300 N up to a deformation of about 2,5 mm. Consequently, you can according to the present invention use lids, housings, sealing rings and welding tools or other tools for the attachment within wide tolerances.

The invention is of course not restricted to the embodiments described as examples above, but may be varied within the limits of the following claims. Details may for example be modified in accordance with corresponding details in the above mentioned patents.

## Claims

1. A seal comprising a ring (17) of a resilient material intended to be pressed between two parallel, preferably flat sealing surfaces (13,16), **characterized** in that the dimension of the ring (17) perpendicular to the two parallel surfaces (13,16) is larger than the dimension parallel to said surfaces and that it is given such a form that it is bent in a certain direction in its sealing position.

2. A seal according to claim 1, **characterized** in that it includes beside said two parallel sealing surfaces (13,16) a third sealing surface (19), which is perpendicular to the other sealing surfaces and encloses the ring (17).

3. A seal according to claim 2, **characterized** in that the transverse section of the ring (17) is given such a form that it is directing a concave surface against said third sealing surface (19) in its sealing position.

4. A seal according to any of the preceding claims, **characterized** in that the transverse section of the ring (17) has the form of a boomerang or a C-form before it is compressed, the concave side of the ring being directed outwardly against said third sealing surface (19).

5. A seal according to claim 4, **characterized** by an indentation (20) on the convex side of the ring, having such a form that said side (21) of the ring will get an essential flat form perpendicular to the two first mentioned sealing surfaces (13,16).

6. A seal according to any of the preceding claims, being a part of a filtration and/or diffusion device, comprising a bundle (1) of hollow fibers arranged longitudinally within a housing (2) with the fiber ends embedded in two end walls (3), **characterized** in that one sealing ring (17) is positioned between the outside of one or both said end walls (3) and a lid (14) having an inlet and/or an outlet (15) for a liquid intended to flow through the hollow fibers of the bundle.

7. A seal according to claim 6, **characterized** in that the lid or lids (14) has/have a cup-form with a flange which covers the outside of the ends of the housing (2).

8. A seal according to claim 7, **characterized** in that the lid (14) is tightly welded or glued to the housing (2).

9. A seal according to claim 7, **characterized** in that the lid (14) has a screw connection (18a) with the housing (2).

10. A seal according to any of the preceding claims, **characterized** in that the resilient ring (17) is made of rubber or a similar elastic material.

11. A seal according to any of the preceding claims, **characterized** in that it is a part of a filtration and/or diffusion device according to any of the Swedish patents    (SE appl 87.03366-8) and 454 847 (SE appl 87.03368-4).

12. A seal comprising a ring (17) of a resilient material intended to be pressed between two parallel, preferably flat sealing surfaces (13-16), **characterized** in that the ring (17) has such a form that it by the compression quickly reaches a knicking or bending position from which the compression forces increases slowly compared with the initial state of the compression, eventually reaching a position from which the compression forces once again increases more rapidly.

## Fig. 1

## Fig. 2

*Fig. 3a*

*Fig. 4a*

17

17'

*Fig. 3b*

*Fig. 4b*

17

21

17'

*Fig. 3c*

*Fig. 4c*

17

20

17'

Fig. 5

European Patent Office

**EUROPEAN SEARCH REPORT**

Application Number

EP 89 11 1819

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | DE-A-1 809 957 (KEMPCHEN & CO.) * Figures 1-3; claims 1,3,7,8; page 5, last paragr aph page 6, last line * | 1-4,12 | F 16 J 15/02 B 01 D 63/02 B 01 D 65/00 |
| Y | | 6-9,11 | |
| X | GB-A-2 187 805 (T.P. NICHOLSON) * Whole document * | 1,4,12 | |
| X | EP-A-0 192 015 (V. SÖDERBERG) * Figures 1-2; abstract; claims 1-3; page 3, line 4-page 4, line 27 * | 1-3,12 | |
| X | EP-A-0 152 212 (AEROQUIP AG) * Figures 2-5; abstract; claim 1; page 1, lines 1-5; page 2, lines 5-10; page 4, line 18- page 7, line 11 * | 1,10,12 | |
| Y | GB-A-2 090 546 (MONSANTO COMP.) * Abstract; figures 5-8; claims 1,3; page 1, line 94- page 2, line 10; page 4, lines 26-51; page 9, lines 62-65; page 9, line 126- page 10, line 35 * | 6 | |
| D,Y | SE-B- 454 847 (GAMBRO)(06-06-1988) * Abstracts; claims 1,10; figures 3,6,9,9a; column 5, lines 39-56 * & EP-A-305 687 (GAMBRO) 08-03-1989 | 6-9,11 | TECHNICAL FIELDS SEARCHED (Int. Cl.5)  B 01 D F 16 J A 61 M |
| P,Y | EP-A-0 305 672 (GAMBRO)(08-03-1989) * Abstract; claim 1; figure 2 * & SE-A-87 3366 (Cat. D) | 6-9,11 | |

The present search report has been drawn up for all claims

| Place of search | Date of completion of the search | Examiner |
|---|---|---|
| THE HAGUE | 24-10-1989 | HOORNAERT P.G.R.J. |